# EUROPEAN PATENT APPLICATION

(11) **EP 2 199 783 A1**
(43) Date of publication of application: **23.06.2010**
(21) Application number: 08171886.8
(22) Date of filing: 17.12.2008
(51) Int. Cl.: G01N 27/02, G01N 33/487, C12M 1/34

(54) **Microelectronic device for measuring cell adhesion**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention relates to a microelectronic device (10) for measuring cell adhesion to a surface. To allow measuring of the adherence of low-coverage, non-confluent cell types, a microelectronic device (10) is proposed comprising:
- a sample chamber (11) for insertion of cells (18),
- an impedance electrode unit (140) comprising an array of impedance electrodes (14) arranged in said sample chamber (11),
- a counter electrode unit (17) comprising at least one counter electrode (17; 171, 172) arranged in said sample chamber (11), and
- an impedance measurement unit (16) for separately measuring impedance measurement signals representing the impedance between said impedance electrodes (14) and said at least one counter electrode (17) over time, the cell adhesion being derived from said impedance measurement signals.

## Description

### FIELD OF THE INVENTION

The present invention relates to a microelectronic device for measuring cell adhesion to a surface.

### BACKGROUND OF THE INVENTION

The adhesion of cells to a surface is an interesting property for a variety of ailments and diseases. For example, for coronary artery disease the adhesion and migration of monocyte cells through the artery wall to a point of inflammation is critical in determining the prognosis for patients with atherosclerotic plaques.

Similarly, the recruitment of monocytes by a tumor is important for the process of angiogenesis, i.e., the creation of new blood vessels to in this case sustain a growing tumor. Blocking of this recruitment is in fact the basis for a new generation of cancer therapies.

In the case of Coronary Artery Disease the endothelial cells at the point of inflammation produce Vascular Endothelial Growth Factor (VEGF). This protein interacts with receptors on circulating monocytes and results in a migration (chemotaxis) of these cells into the artery wall at the point of inflammation. It has recently been suggested that the mobility of monocytes isolated from the blood of patients could be used as a bio-marker for patient risk stratification and for forming a prognosis for the disease.

Measuring the chemotaxis response of monocytes is a time consuming process. A gradient of VEGF has to be formed and then the movement of many cells monitored in order to build up sufficient statistics. In the case of monocyte migration this movement is particularly slow and it may take hours before significant movement can be recorded. It would therefore be beneficial to be able to measure parameters which can give an earlier indication of monocyte response, such as adhesion. Adhesion can be measured much faster than cell migration (minutes instead of hours). Adhesion is the first step in the process towards chemotaxis and it has recently been shown that adhesion is up-regulated in monocytes which have been exposed to ac-LDL. This is significant as ac-LDL is contained within atherosclerotic plaques (atheromas).

The adhesion of cells can be measured using electrical impedance. This is a very convenient method as it is label free and yields quantitative results. There are instruments on the market from Applied Biophysics and Acea Biosciences which can be used for measuring this. The cartridges for these instruments consist of multiple wells, comprising in each of these wells a central electrode, and an annular electrode around this electrode. Such devices are also disclosed in US 2005/0130119 A1 and WO 2004/010102 A2.

The central electrode is known as the active electrode. As cells start to adhere to this the impedance of the central electrode changes. This is due to two reasons:
(i) the volume under the cell becomes less as the cell adherence increases which results in a changing in capacitance and
(ii) as the cells grow towards one another the area of the electrode which is not covered decreases and hence the low resistance paths between the cells decreases .

Another known electrode design shows still only one central electrode but with an insulator on top. An array of holes is created in the insulator. In this case the exposed electrode at each hole is connected together by the underlying metal and is therefore not individually addressable.

When monocytes come into contact with a surface then they quickly differentiate into macrophages. Macrophages are "sticky" and quickly adhere to the active electrode. They do not, however, form a, continuous, confluent layer and therefore the largest fraction of the electrode is left exposed. In some cases only 10-15% of the electrode is actually covered by the cells.

This low coverage is a major problem with the known devices.

An additional problem in the absence of a confluent monolayer is that the number of cells located on the active electrode often determines the impedance rather than the actual adhesion of the present cells. The number of cells on the electrode surface is difficult to control and can vary significantly from one measurement to another. The cells on the surface could be counted (e.g. by inspecting the electrode with a microscope) to normalize the measured impedance, but this procedure is time consuming and is not applied in practice.

### SUMMARY OF THE INVENTION

It is an object of the present invention to provide an improved microelectronic device which allows measurement of the adherence of low-coverage, non-confluent cell types.

According to the present invention a microelectronic device for measuring cell adhesion to a surface is presented comprising:
- a sample chamber for insertion of cells,
- an impedance electrode unit comprising an array of impedance electrodes arranged in said sample chamber,
- a counter electrode unit comprising at least one counter electrode arranged in said sample chamber, and
- an impedance measurement unit for separately measuring impedance measurement signals representing the impedance between said impedance electrodes and said at least one counter electrode over time, the cell adhesion being derived from said impedance measurement signals
   Preferred embodiments of the invention are defined in the dependent claims.
   The present invention is based on the idea to address the problem of measuring low density non-confluent cell types by a novel electrode structure. The impedance electrode unit comprises, according to the present invention, a segmented electrode, i.e. a plurality of impedance electrodes. For said impedance electrodes separate impedance measurement signals are obtained over time by an appropriate impedance measurement unit. Such an impedance measurement unit and the principle of impedance measurement are generally known per se. From the obtained impedance measurement signals information about the adhesion of the cells to the impedance electrodes can be derived then, in particular information about the strength and/or rate of adhesion. As explained above the impedance between a particular impedance electrode and the counter electrode increases if cells adhere to the electrode, so that the impedance measurement signal for a particular impedance electrode allows to retrieve information about the adhesion of cells to this particular impedance electrode.
   According to a preferred embodiment the microelectronic device further comprises a processing unit adapted to
- process said impedance measurement signals;
- determine an average impedance value from said impedance measurement signals, and
- update said average impedance value over time by disregarding impedance measurement signals, which are substantially constant over time, for the determination of the average impedance value.

Thus, according to this embodiment it is observed if the signal level of impedance measurement signals changes. These impedance measurement signals are then used for determining the running average. In this way the impedance measurement signal amplitude related to adhesion of cells can be increased and a reduction in noise can be achieved.

Preferably, the signal variations after adhesion are logged in order to quantify the strength of adhesion since fewer variations imply a stronger adhesion. It should be noted that this is different from the rate of adhesion.

Advantageously, the processing unit is adapted for checking if the most recent impedance signal is constant over time with respect to the amplitude of the impedance measurement signal of the same impedance electrode at a predetermined moment in time or at a predetermined condition. Thus, the step of checking takes as a reference a certain moment in time, e.g. when the cells are added into the sample chamber, or a certain condition, e.g. when only cell medium (but no cells) is present in the sample chamber.

While the impedance electrodes could be one- or two-dimensional in some way, they are preferably arranged as an array. This makes addressing and impedance measurement more simple since known ways of addressing, using e.g. row and column addressing means, can be used.

For the counter electrode unit different embodiments can be envisaged. According to a first embodiment said at least one counter electrode is arranged remotely from said array of impedance electrodes. According to a second embodiment said at least one counter electrode is dispersed, as a kind of grid electrode, between the impedance electrodes, in particular arranged in gaps between neighboring impedance electrodes. The advantage of a remote arrangement of the counter electrode over a grid counter electrode is mainly that the impedance electrodes can be closer together which gives less dead area between them. Grid counter electrodes, in contrast, are more sensitive, prevent cross talk and provide that all impedance electrodes are equivalent.

Further, said at least one counter electrode unit comprises preferably a plurality of counter electrodes to improve the sensitivity of impedance measurement.

In an advantageous embodiment the impedance electrodes are separately surrounded by a separate first counter electrode and one or more separate second counter electrodes are arranged in gaps between neighboring first counter electrodes. Such an arrangement allows for the use of alignment signals for aligning cells above the impedance electrodes.

It has been found that an optimal grid width geometry is that the size of the grid electrode is less than the width of cell. For instance, in a practical implementation it has been found that a grid width of about 7 µm and gaps having a width of about 7 µm is ideal.

As mentioned already, a preferred embodiment further comprises cell alignment means for aligning cells with respect to said impedance electrodes. These can be used to align single cells or groups of cells per impedance electrode. This improves the efficiency of measurement since interference is avoided and cell viability is ensured.

Preferably, said cell alignment means are adapted for controlling said impedance measurement unit to generate an AC signal to be provided to said impedance electrodes and for sweeping an impedance measurement signal. Depending on the electrode geometry (i.e. if the counter is a grid) then the alignment signal can be applied to the same impedance electrodes that are used for the impedance measurement. Thus, no separate electrodes are required.

According to a different embodiment said cell alignment means are coupled to said impedance electrode unit and said counter electrode unit and are adapted for generating dielectrophoresis (DEP) signals and for providing a high field region above impedance electrodes at which no cells shall be located. These DEP signal can be used and adapted such that cells are pushed away from impedance electrodes where no cells are wanted.

The cells adhered to impedance electrodes can be counted optically, for instance to normalize the impedance measurement signal. For this purpose it is advantageously proposed that said measurement electrodes are transparent and that said microelectronic device further comprises optical detection means, in particular a micro-camera or a photodiode for counting the number of cells adhered to single, a number of or all impedance electrodes and/or for measuring the fraction of single, a number of or all impedance electrodes covered by cells. They are preferably located, in particular if photodiodes are used, underneath the transparent impedance electrodes, i.e. on the same side of a substrate as the adhering cells. However, the optical detection means can also be located on the opposite side of the impedance electrodes, i.e. the side opposite the side to which the cells shall adhere.

For normalizing an impedance measurement signal by use of optical detection signals of said optical detection means signal normalizing means are optionally provided.

Preferably, the impedance electrodes are provided in LTPS technology. In an embodiment the microelectronic device comprises a plurality of wells including electrically active components and integrated circuitry for individual addressing of said wells or active components thereof. For instance 96 or 384 wells can be provided in a practical implementation. The electrically active components may include electrodes for impedance sensing/positioning, photodiodes, thin film heaters and sensors, and/or fluidic pumps.

### BRIEF DESCRIPTION OF THE DRAWINGS

These and other aspects of the invention will be apparent from and elucidated with reference to the embodiment(s) described hereinafter. In the following drawings
Fig. 1 shows schematically a first embodiment of the microelectronic device according to the present invention,
Fig. 2 shows a top view on the first embodiment,
Fig. 3a shows a top view on the first embodiment with adhering cells,
Fig. 3b shows impedance measurement signals for the first embodiment shown Fig. 3a,
Fig. 4 shows a top view on a second embodiment of the microelectronic device according to the present invention,
Fig. 5 shows a top view on a third embodiment of the microelectronic device according to the present invention,
Fig. 6 shows schematically a fourth embodiment of the microelectronic device according to the present invention,
Fig. 7 shows schematically a fifth embodiment of the microelectronic device according to the present invention, and
Fig. 8 shows schematically a sixth embodiment of the microelectronic device according to the present invention.

### DETAILED DESCRIPTION OF THE INVENTION

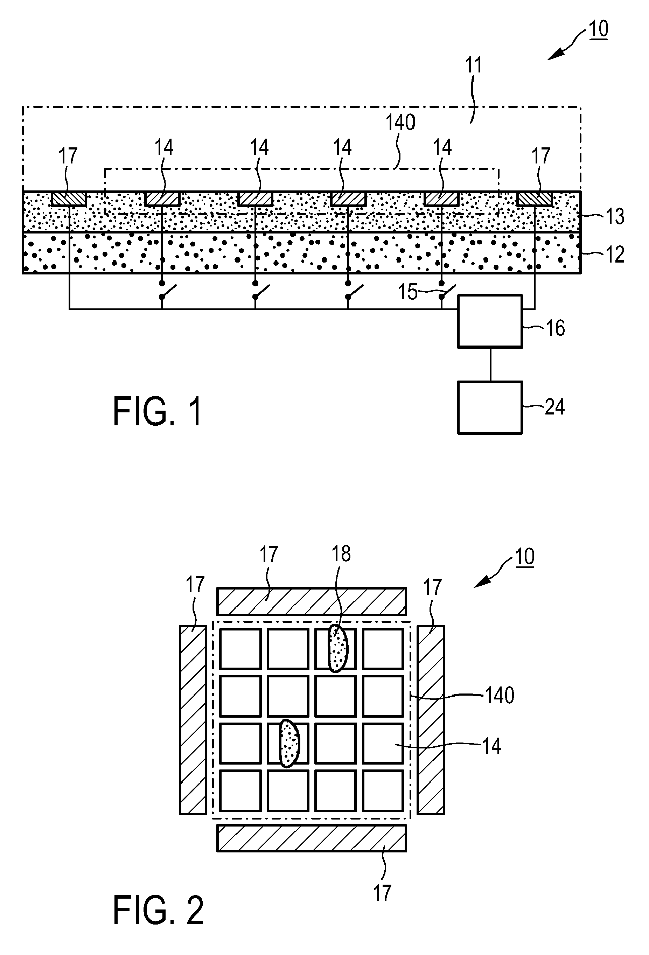

Fig. 1 shows a schematic cross section through an embodiment of a microelectronic device 10 according to the present invention. The device 10 comprises a sample chamber 11 in which a sample to be investigated can be provided. Moreover, it comprises a substrate 12 (e.g. a glass plate) which constitutes the bottom wall of the sample chamber 11. The interface 13 between said substrate 12 and the sample chamber 11 is preferably coated with binding sites (not shown) to which target molecules of a sample (optionally labeled with detectable markers) can specifically bind.

It should be noted that the coating of the substrate 12 with adhesion molecules is possible, but it is not per se necessary. In other embodiments the substrate 12 and/or the interface 13 (and/or even the electrodes 14 themselves) are coated only with fibronectin or cologne. It is also possible that the electrodes 14 can be coated with molecules that are related to promotion hindrance of adhesion, such as VCAM (vascular cell adhesion molecule), VEGF (vascular endothelial growth factor), LDL (low density lipoprotein) etc..

A one- or two-dimensional array of impedance electrodes 14 (forming an impedance electrode unit 140) is disposed on the substrate 12, which are coupled via switches 15 to an impedance measurement unit 16 such that they can be individually addressed. When a large number of impedance electrodes 14 are used, conventional large area electronics, as known, for instance, from the display technology should be used to allow individual addressing of the impedance electrodes 14 without an excessively large number of connections to the outside world. These technologies, often based on cheap glass substrates, include amorphous or low temperature silicon. More details about these technologies can be found in WO 2007/034374 A2.

The impedance measurement unit 16 is further connected to a counter electrode 17 to measure the impedance between said impedance electrodes 14 and said counter electrode 17 in a known manner using known circuitry and methods.

Means and circuits for impedance measurement are generally known in the art. Those known means can be applied here. Particular embodiments, which can be applied here, are described in WO 2007/107892 A1, the description of which is herein incorporated by reference.

Fig. 2 shows a top view of the first embodiment of the microelectronic device 10 shown in Fig. 1. The simplest solution to allow the measurement of non-confluent cell types 18 is to segment the central impedance electrode unit 140 into electrode segments 14 and measure the impedance of these impedance electrodes 14 separately over time. In this embodiment the counter electrode 17 is situated remotely from the central array 140 of impedance electrodes 14.

When measuring with such a device then the impedance of all impedance electrodes 14 is measured. If, however, the impedance of one of the impedance electrodes 14 starts to significantly increase with respect to the value at t=0 s then this is a trigger that the impedance of this impedance electrode 14 should be added to the running average as proposed according to a preferred embodiment of the present invention. For this purpose, a processing unit 24 is provided according to this embodiment coupled to the impedance measurement unit 16 for processing said impedance measurement signals. When, however, the impedance of an impedance electrode 14 is approximately constant (if cells are viable then will always see some "noise" due to cell activity) then this is disregarded from the running average. This embodiment has three advantages when measuring non-adherent cells: Increase in signal related to cell adhesion: Since the signal from impedance electrodes 14 which are not covered by cells 18 is disregarded then the signal containing information about adhesion and how this evolves is dominant.

Reduction in noise: Since impedance electrodes 14 where no cells are present are disregarded then the noise from these impedance electrodes 14 is also disregarded.

Since the impedance electrodes 14 counted always have a cell adherent then this decouples the measured impedance from the parameter of electrode coverage which can dominate the impedance signal at the low seeding densities normally used for non-adherent cells. In fact, the electrode coverage parameter is obtained as an independent parameter and can also be used for characterization purposes.

In general, reducing the size of the area of a single impedance electrode (and dividing it into a plurality of impedance electrodes) is beneficial for the sensitivity of the measurement, since it leads to a higher concentration of field lines at the probing surface.

According to a slightly modified embodiment, instead of the impedance signal at t=0 also the value measured when only cell medium (and no cells) is present in the sample chamber 11) could be used as a reference. This might allow also detecting cells 18 that are deposited on an impedance electrode 14 but do not adhere over time.

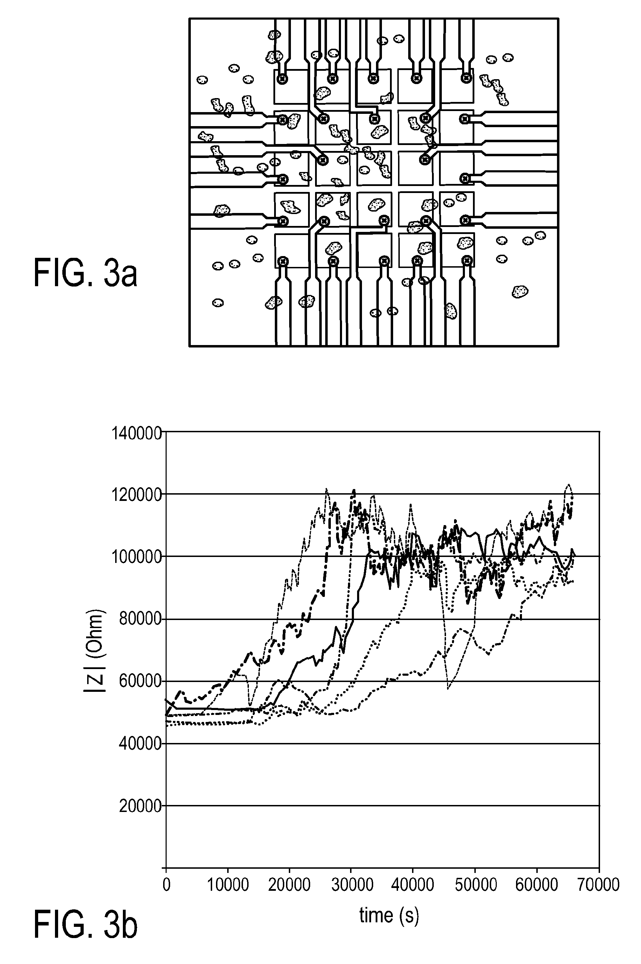

Fig. 3a shows a proof of the principle with adhering cells 18 on a impedance electrode unit 140 in the form or an electrode array comprising 25 impedance electrodes 14. Fig. 3b shows the corresponding impedance curves for various impedance electrodes. To maximize the signal-to-noise ratio each electrode (impedance) signal should be excluded from the running average when the impedance is above a predetermined and appropriate (for the cell type and electrode number and geometry) threshold, for instance 100kohm.(for the particular cell type shown in Fig 3(a,b) and measured on this particular electrode geometry).

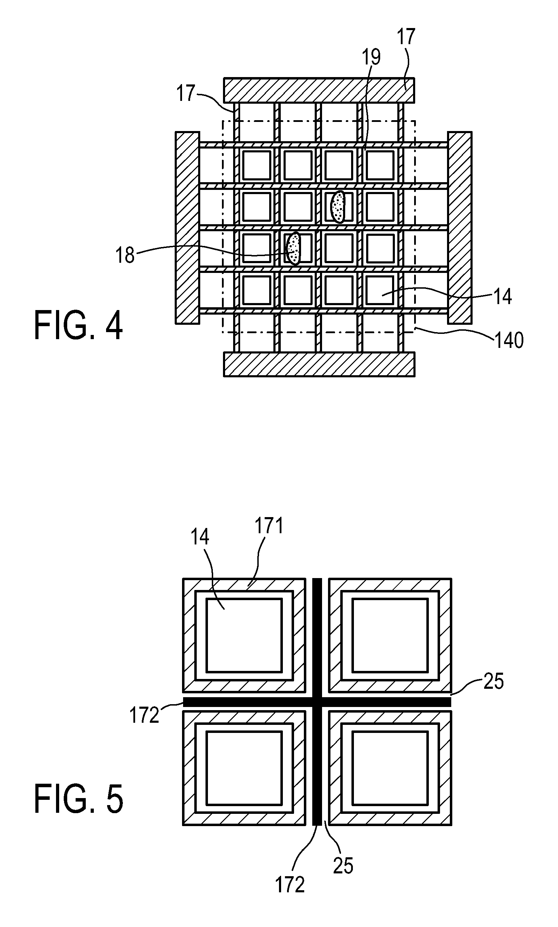

According to another preferred embodiment the counter electrode 17 is not remote but instead is dispersed in gaps 19 between the impedance electrodes 14 as shown in Fig. 4. This has three advantages: (i) it is more sensitive, (ii) the measurement field is localized to the pixel that it is applied. i.e. the ground acts as an electrical guard so as to prevent the cell coverage of neighboring impedance electrodes 14 contributing to the impedance signal (cross talk). (iii) All impedance electrodes 14 in the array 140 are equivalent.

In still another embodiment illustrated in Fig. 5 there is not just one counter electrode but a multitude of counter electrodes 171, 172. According to this embodiment the impedance electrodes 14 are separately surrounded by a separate first counter electrode 171 and one or more separate second counter electrodes 172 are arranged in gaps 25 between neighboring first counter electrodes 171. This design allows for an alignment signal to be applied between grids (at the second counter electrodes 172) while at the same time allowing the impedance to be measured between the inner grid (first counter electrode 171) and the impedance electrode. as will be explained below,

It is possible to use the impedance electrodes for measuring impedance but also to manipulate cells before attachment and so allow a certain cell pattern. This is done by the method of dielectrophoresis (DEP) which can result in aligning one cell per pixel or a group of cells together. This could be used to increase the efficiency of the measurement by ensuring that each cell present is aligned with an impedance electrode, or to ensure that single cells are separated by a sufficient space to avoid interference and ensure cell viability. To achieve DEP alignment the dispersed counter electrode design as shown in Fig. 4 is preferably used. In this design there is automatically an electrical field created between the impedance electrodes 14 and the counter electrode 17 during the measurement. Given the high conductivity of culture medium this will force the cells 18 away from the gap in-between the impedance electrodes 14 and result in a cell measurement-electrode alignment. With high conductivity dielectrophoresis is negative (due to the conductivity difference between the cell components and the medium). Negative DEP forces the cells away from the gap.

In theory the use of the measurement signal to both measure and align cells is possible. This is however not ideal because; (i) the measurement signal is usually much lower than that required for DEP, (ii) the frequency is swept for the impedance measurement which means the alignment force is not constant and (iii) there is no force on a pixel if the impedance is read out sequentially.

According to still another embodiment it is therefore proposed to superimpose a predetermined frequency of electrical field on all pixels such as to create the alignment force and at the same time sweep the impedance measurement signal. A filter can be used to eliminate the alignment force frequency from the impedance sweep signal. Alternatively a phase filter can be used for signal separation.

DEP signals, which can be used for this purpose, are a sine wave of, e.g. 100kHz having an amplitude in the range of 1-5 V. If the impedance electrode oscillates with the same phase as the counter electrode then there is no field above the impedance electrode. If it oscillates with a 180° phase difference then it is a high field area above the impedance electrode. For mammalian cells in growth medium then the cells will be pushed towards the low field region. To locate a cell above an impedance electrode then all other impedance electrodes preferably have a phase difference and the impedance electrode where they are required should oscillate with the same phase

Preferably, for alignment with the dispersed counter electrode, as shown in Fig. 4, the electrode grid width should be less that the diameter of a cell. For example, a 7 µm electrode grid with 7 µm gaps has been found to be ideal.

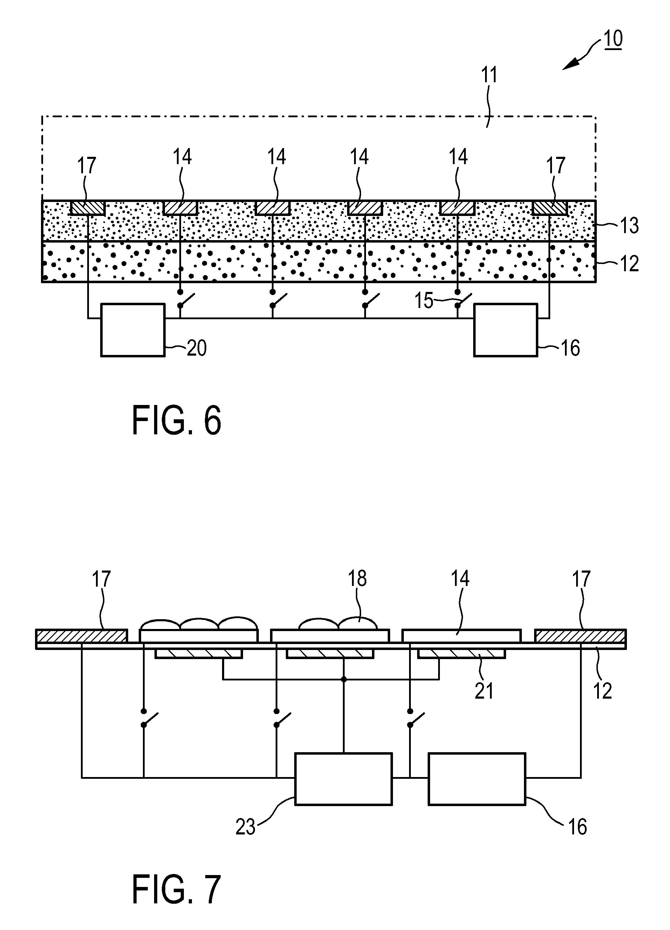

A possible approach proposed according to preferred embodiment is to apply a DEP voltage signal (typically 1-5V) to impedance electrodes 14 where no cell is wanted. A cell alignment unit 20 as shown in the embodiment of the microelectronic device 10 depicted in Fig. 6 can be used for this purpose The cells will then be pushed towards impedance electrodes 14 which are not activated. A signal at a much lower voltage (mV) and with a variable frequency can then be applied to latter electrodes 14 to read out the impedance signal. As the voltage amplitude for impedance is much lower than that required for DEP, the impedance measurement will not interfere with cell adhesion.

DEP can also be used to exert a repulsive force on adhered cells to detach them and follow the restoration of adhesion over time. For this purpose, a DEP voltage is applied by the cell alignment unit 20 at regular intervals to impedance electrodes where cells have been detected by impedance. The voltage at which the cells detach could also be used as a measure of the strength with which a cell is attached to the impedance electrode.

Preferably, the array 140 of impedance electrodes 14 and the counter electrode 17 can be provided in LTPS (Low Temperature Poly Silicon) technology. For instance, an LTPS substrate is present where components such as transistors, diodes and heaters can be integrated onto the substrate. Details about the LTPS technology, which can be applied according to the present invention, are described, for instance, in the above mentioned WO 2007/034374 A2.

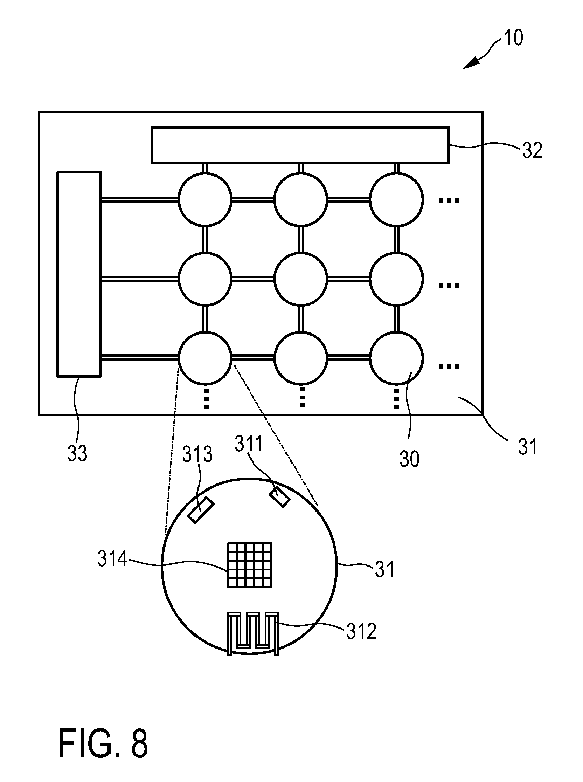

In an embodiment, as shown in Fig. 8, the microelectronic device 10 comprises a plurality of (fluidic) wells 30 (only 9 out of, e.g., 96 wells are shown) on a substrate 31 including electrically active components and integrated circuitry, in particular shift registers 32 for columns and shift registers 33 for rows of wells for individual addressing of said wells or active components thereof. For instance, 96 or 384 wells can be provided in a practical implementation. The electrically active components may include electrodes/circuitry 311 for impedance sensing/positioning, photodiodes, thin film heaters 312, sensors 313 (e.g. for pH or O₂), an impedance array 314 for measuring adherence and/or fluidic pumps.

An embodiment of a typical circuit for a heater 312 is, for instance, described in WO 2007/107892 A1.

The present invention is mainly directed to measuring the adhesion of non-confluent cell types. This effectively means that there is a low numbers of cells present on the impedance electrode(s) i.e. each impedance electrode can be covered with a different number of cells. When the impedance signal is measured then the signal can either be determined by just the number of cells that are present on the impedance electrodes or the adhesion of these cells. It is therefore advantageous, as proposed according to a further embodiment, to measure not only the impedance but also make an (optical) image to be able to normalize the impedance signal with the number of cells present. To be able to do this the sample can be placed under a an optical imaging device, such as a camera or a microscope, which, however, makes the set-up more complicated and expensive.

In a preferred embodiment an optical detection means 21, in particular a micro-camera or a photodiode is placed via LTPS on the substrate 12 directly under a transparent impedance electrode 14 as shown in Fig. 7. The optical detection means 21 provide optical detection signals indicating the fraction of the impedance electrode 14 which is covered by cells 18 and the (above described) impedance signal provides information about the adhesion. The optical detection signals can be used in a signal normalizing means 23 to normalize the impedance measurement signals of the respective impedance electrodes 14.

It shall be noted that the optical detection means 21 can also be located in between the transparent impedance electrodes 14 and the substrate 12.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measured cannot be used to advantage.

Any reference signs in the claims should not be construed as limiting the scope.

## Claims

1. Microelectronic device (10) for measuring cell adhesion to a surface, comprising:
- a sample chamber (11) for insertion of cells (18),
- an impedance electrode unit (140) comprising an array of impedance electrodes (14) arranged in said sample chamber (11),
- a counter electrode unit (17) comprising at least one counter electrode (17; 171, 172) arranged in said sample chamber (11), and
- an impedance measurement unit (16) for separately measuring impedance measurement signals representing the impedance between said impedance electrodes (14) and said at least one counter electrode (17) over time, the cell adhesion being derived from said impedance measurement signals.

2. Microelectronic device as claimed in claim 1,
further comprising a processing unit (24) adapted to
- process said impedance measurement signals;
- determine an average impedance value from said impedance measurement signals, and
- update said average impedance value over time by disregarding impedance measurement signals, which are substantially constant over time, for the determination of the average impedance value.

3. Microelectronic device as claimed in claim 2,
wherein said processing unit (24) is adapted for checking if the most recent impedance measurement signal is constant over time with respect to the amplitude of the impedance measurement signal of the same impedance electrode (14) at a predetermined moment in time or at a predetermined condition.

4. Microelectronic device as claimed in claim 1,
wherein said impedance electrodes (14) are arranged as an array (140).

5. Microelectronic device as claimed in claim 4,
wherein said at least one counter electrode (17) is arranged remotely from said array of impedance electrodes (14).

6. Microelectronic device as claimed in claim 4,
wherein said at least one counter electrode (17) is dispersed between the impedance electrodes (14), in particular arranged in gaps (19) between neighboring impedance electrodes (14).

7. Microelectronic device as claimed in claim 4,
wherein said at least one counter electrode unit comprises a plurality of counter electrodes (171, 172).

8. Microelectronic device as claimed in claim 7,
wherein said impedance electrodes (14) are separately surrounded by a separate first counter electrode (171) and wherein one or more separate second counter electrodes (172) are arranged in gaps (25) between neighboring first counter electrodes (14).

9. Microelectronic device as claimed in claim 4,
wherein the grid width of said array of impedance measurement electrodes (14) is smaller than the diameter of cells (18) whose adhesion shall be measured.

10. Microelectronic device as claimed in claim 4,
further comprising cell alignment means (20) for aligning cells (18) with respect to said impedance electrodes (14).

11. Microelectronic device as claimed in claim 10,
wherein said cell alignment means (20) are adapted for controlling said impedance measurement unit (16) to generate an AC signal to be provided to said impedance electrodes (14) and for sweeping an impedance measurement signal.

12. Microelectronic device as claimed in claim 10,
wherein said cell alignment means (16) are coupled to said impedance electrode unit (140) and said counter electrode unit (17; 171, 172) and are adapted for generating dielectrophoresis (DEP) signals and for providing a high field region above impedance electrodes (14) at which no cells shall be located.

13. Microelectronic device as claimed in claim 1,
wherein said impedance electrodes (14) are transparent and
wherein said microelectronic device further comprises optical detection means (21), in particular a micro-camera or a photodiode, for counting the number of cells adhered to single, a number of or all impedance electrodes (14) and/or for measuring the fraction of single, a number of or all impedance electrodes (14) covered by cells (18).

14. Microelectronic device as claimed in claim 13,
further comprising signal normalizing means (23) for normalizing impedance measurement signals by use of optical detection signals of said optical detection means (21).

15. Microelectronic device as claimed in claim 1, comprising a plurality of wells including electrically active components and integrated circuitry for individual addressing of said wells or active components thereof.
